(19) **Europäisches Patentamt European Patent Office Office européen des brevets**

(11) **EP 0 847 265 B2**

(12) **NEW EUROPEAN PATENT SPECIFICATION**
After opposition procedure

(45) Date of publication and mention
of the opposition decision:
**12.10.2011 Bulletin 2011/41**

(45) Mention of the grant of the patent:
**20.03.2002 Bulletin 2002/12**

(21) Application number: **96930556.4**

(22) Date of filing: **20.08.1996**

(51) Int Cl.:
***A61F 13/15*** *(2006.01)*

(86) International application number:
**PCT/US1996/013487**

(87) International publication number:
**WO 1997/009015 (13.03.1997 Gazette 1997/12)**

(54) **ABSORBENT ARTICLE HAVING A STABILIZING ABSORBENT ELEMENT**

ABSORBIERENDER ARTIKEL MIT EINEM SAUGFÄHIGEN VERSTÄRKUNGSELEMENT

ARTICLE ABSORBANT A ELEMENT ABSORBANT STABILISANT

(84) Designated Contracting States:
**AT BE CH DE ES FR GB GR IE IT LI NL SE**

(30) Priority: **01.09.1995 US 522881**

(43) Date of publication of application:
**17.06.1998 Bulletin 1998/25**

(73) Proprietor: **McNeil-PPC, Inc.
Skillman,
New Jersey 08558 (US)**

(72) Inventors:
• **SALERNO, Catherine, E.**
**Malabar, NSW 2036 (AU)**
• **JERSCHKOW, Tenny**
**Middletown, NJ 07748 (US)**
• **GENTILE, Michele**
**Port Reading, NJ 07064 (US)**

(74) Representative: **Mercer, Christopher Paul
Carpmaels & Ransford
One Southampton Row
London
WC1B 5HA (GB)**

(56) References cited:
**EP-A- 0 335 253    EP-A1- 0 570 016
WO-A-96/12460    GB-A- 2 055 586
US-A- 3 375 827    US-A- 4 195 634
US-A- 4 806 408    US-A- 4 936 839**

EP 0 847 265 B2

**Description**

**Field of the Invention**

The present invention relates to an absorbent article for absorbing body fluids such as menstrual fluid, vaginal discharge and/or urine, and more particularly, to an absorbent article having a stabilizing element which is resistant to wet collapse, and provides the article with enhanced resistance to bunching and inward collapse due to lateral compressive forces imparted by a user's thighs so that a central absorbent portion of the article maintains contact with the perineal area of the user and thus reduces the probability of leakage and failure.

**Background of the Invention**

Conventional full-size sanitary protection and feminine hygiene products such as sanitary napkins, adult incontinence devices, etc., typically contain an absorbent element, a fluid-pervious body-contacting element and a fluid-impervious undergarment-facing element. These articles are intended to absorb body fluid from the wearer and retain the fluid in order to prevent the fluid from soiling the wearer's garments. Unfortunately, conventional feminine hygiene articles do not adequately fulfill women's protection requirements. Sanitary napkins generally have the capability of absorbing between 50 and 100 grams of fluid. However, soiling of a wearer's garments still occurs even when only 5 to 10 grams of fluid has been deposited on the absorbent articles. One of the primary reasons why soiling occurs is that conventional napkins do not properly conform to the perineal area of a woman's body. When there is a space between the user and the pad, body fluid exudate tends to travel along the contours of the body and results in soiling of the undergarment.

One factor that contributes to poor fit and lack of conformity is napkin instability. When a compression force is applied to a sanitary napkin (e.g., by the lateral compressive forces of a user's thighs), the napkin tends to fold or bunch resulting in a smaller area of contact with the user's body resulting in a gap between the absorbent article and the user's body. The napkin center may also become depressed, i.e., move in a direction downward and away from the user's body. Fluid may then travel along the body and bypass the napkin resulting in soiling of the undergarment.

An additional factor that contributes to poor fit centers on the misconception that the outer genital area of females, longitudinally between the thighs in the areas of the urethral and vaginal openings, is curved; when in fact it is essentially flat or planar. Thus, there has been a tendency on the part of some inventors to develop curved products, wrongly assuming that the centermost portions of such a product will fit closely to the body.

For example, European Patent No. 091,412 depicts an absorbent product having an absorbent core which is convex in the center and has elasticized side margins. U.S. Patent No. 4,770,657 to Ellis also describes a curved sanitary napkin with elasticized side edges.

Another example of a curved sanitary napkin is illustrated in WO 88/04547 to Thoren which discloses a design in which elastic means are prestretched. This causes the napkin to be resiliently distorted so that the sides assume a convex shape when held against the user's body. Upon application of pressure, the shape will flatten out. However, when the pressure is reduced or eliminated, the napkin resumes its curved shape. Further this design lacks a structural element and, therefore, when wetted, will collapse and move away from the user and cause soiling of the undergarment.

Several prior art patents discuss absorbent systems which attempt to address the soiling problem in different ways. One method is to create a resilient and/or stabilized absorbent system in order to prevent the napkin from bunching when worn. For example, U.S. Patent No. 4,195,634 to DiSalvo describes a "stiffener means" which is incorporated into the napkin and positioned between the absorbent core and the barrier along the entire length of the pad to resist side compression. However, this design is deficient in that the stiffening/stabilizing element is located below the absorbent medium. The absorbent medium tends to collapse when exposed to fluid, causing it to move away from the wearer, despite the presence of the stiffener means. Furthermore, since the stiffener means is not conformable, it will not adapt to the body and will be uncomfortable to wear.

U.S. Patent No. 4,405,326 to Lengham describes a design which is similarly deficient. U.S. Patent No. 4,217,901 to Bradstreet also describes a crush-resistant sanitary napkin, but does not specifically address the issue of body-fit.

European Patent Applications 0 335 252 and 0 335 253 to Buell which forms the preamble describe a disposable absorbent article having a flexure-resistant, deformation element that is not moisture-sensitive. The deformation element has a convex upward configuration when the napkin is worn and pressed inward by the thighs. The proposed design relies on the lateral compressive forces of the wearer's thighs to form a convex upward configuration. However, this design is also flawed with respect to maintaining body-fit and conformation since the convex configuration cannot consistently be controlled by thigh movement, as the napkin's ability to conform to the body relies heavily upon placement of the absorbent article and the wearer's anatomy. Furthermore, there is no means to insure that the absorbent article will stay in place at the appropriate portion of the anatomy. In addition, if the deformation element is not moisture-sensitive, the deformation element cannot be placed near the pad's surface because it cannot absorb fluid. Thus, the absorbent material in the pad which is closer to the body than the deformation element will tend to collapse when exposed to fluid, and move away from the body.

Other attempts to address the problems of bunching and absorbent collapse have suggested an increase in the thickness of the central portion of the absorbent element. Others suggest interlabial products that have a portion fitting between the labia, close to the vaginal orifice. For example, U.S. Patent No. 4,490,147 to Pierce describes a sanitary napkin having a raised center with absorbent pads which are arranged parallel to one another in a pyramid-shaped bundle. The pads are movable with respect to one another and are encased by a liquid pervious cover material. However, such a napkin will not conform to the perineal cavity, particularly at the front and back. The pyramid shape may also cause fluid to roll off the napkin's edge and cause soiling of the wearer's undergarment.

U.S. Patents Nos. 4,631,062 and 4,804,380 to Lassen generally describe self-conforming napkins for partial labial disposition. The napkin contains a posterior region, including a raised profile for placement intermediate the wearer's labia majora and a flattened front portion for placement exterior of the clitoris and pubic mons. However, these products would tend to move during wear and can easily become dislodged from the vestibule. Furthermore, interlabial napkins tend to be uncomfortable to wear for most women.

Another raised center napkin design is described in U.S. Patent No. 2,662,527 to Jacks which discloses an absorbent pad having a main body member of absorbent material and a second absorbent material secured on the face of the main member. The second absorbent material is narrower and shorter than the main member and has dimensions designed to allow it to fit between the labia. Such a design would tend to collapse when exposed to fluid and would be extremely likely to move in relation to the body of the wearer.

Similarly, a napkin described in U.S. Patent No. 3,406,689 to Hicks, has a dual discrete layer system in which the two pads are separated from one another and are freely movable with respect to each other. The two pads are installed separately, would tend to move and are extremely inconvenient. Fluid may also travel from one pad to the edge of the other, resulting in staining the wearer's undergarment. U.S. Patent No. 4,433,972 to Malfitano also contains two pads, although the top pad is hourglass in shape.

U.S. Patent No. 4,425,130 to Desmarais describes a sanitary napkin having a primary menstrual absorbent pad and a "panty protector" which are joined. Such a design has the same types of deficiencies as those described above with regard to compound pads. For example, the panty protector member is intended to protect the user's garments from being soiled by fluids which are expelled from the primary menstrual pad or which inadvertently pass the primary pad. However, this design may encourage fluid to wick to the edge of the panty protector and transfer to the user's garment. It is also possible for the panty protector to fold in and contact the face of the primary menstrual pad. Fluid may then transfer to the user's panty if the panty folds up and around the edges of the panty protector, especially if the panty protector is thin.

Patent publication No. WO 92/07535 to Visscher and Osborn discloses a liquid pervious spacing structure which moves the top body-facing cover sheet away from the absorbent core of the napkin. The spacing structure has an uncompressed and a compressed configuration and an upper and lower portion. However, the components of this construction are unlikely to remain separated when exposed to fluid, due to wet-collapse.

Other products which are designed to protect undergarments from staining with body fluid are those having side extensions that can wrap around a panty crotch. Examples of such products are described in U.S. Patent No. 3,397,697 to Rickard, U.S. Patent No. 4,285,343 to McNair, U.S. Patents Nos. 4,589,876 and 4,687,478 to Van Tilburg and EP Publication No. 0426235 to Osborn. Yet other examples of such designs are described in U.S. Patent No. 4,608,407 to Mattingly, U.S. Patent No. 4,911,701 to Mavinkurve and U.S. Patent No. 4,900,320 to McCoy. However, these references disclose methods for inhibiting undergarment staining which do not involve napkin fit and conformability.

Fluid migration along the body is a key element of soiling. Fluid migration generally occurs when fluid is not immediately absorbed into an absorbent article. Accordingly, the better an absorbent article can maintain contact with the body, the less likely that the fluid will leak or migrate away from the absorbent article and cause soiling.

## SUMMARY OF THE INVENTION

It is an object of this invention to provide an absorbent article, such as a sanitary napkin, which is capable of conforming to the body and capturing fluid as it leaves the body.

It is another object of this invention to provide an absorbent article which is resistant to lateral compressive forces which may be imparted by a user's thighs.

Yet another object of this invention is to provide an absorbent article which is comfortable, resilient, resistant to wet collapse, yet highly absorbent.

In accordance with the present invention, there has been provided an absorbent article as defined in claim 1. Preferred embodiments of the present invention are defined in dependent claims.

## BRIEF DESCRIPTION OF THE DRAWINGS

Figure 1 shows the absorbent article of this invention in plan view from the body-facing side of the article.

Figure 2 is a cross-sectional view of the absorbent article shown in Figure 1 taken along the line 2-2.

Figure 3 is a cross-sectional view of the absorbent article shown in Figure 1 taken along the line 3-3.

## DETAILED DESCRIPTION OF THE INVENTION

The present invention is directed to an absorbent article as defined in claim 1 which has a relatively thick fluid absorbent core, i.e. at least 0.20 inches (0.508 cm) thick, and an absorbent stabilizing element having a lateral width in a range of from at least 0.5 inches (1.27 cm) to less than 1.75 inches (4.445 cm). It is considered an important feature of the present invention that the stabilizing element have a lateral width in the above critical dimensions. More specifically, it has now been found that the use of an absorbent stabilizing element with these dimensions, in combination with a relatively thick absorbent pulp fluff core, provides enhanced resistance to bunching and inward collapse due to lateral compressive forces imparted by a user's thighs.

When placed in an undergarment, the absorbent article of this invention provides a stable, resilient absorbent portion adjacent the labia majora and the vaginal orifice and will permit the central portion of the absorbent article to maintain an intimate fit against the labia majora. The stabilizing element provides resilience and resistance to wet-collapse in the area where it is needed, i.e., near the vaginal orifice or urethra, where the body fluid exits the user and impinges on the absorbent article.

Thus, the absorbent articles of this invention provide enhanced leakage protection due to better body fit without sacrificing comfort.

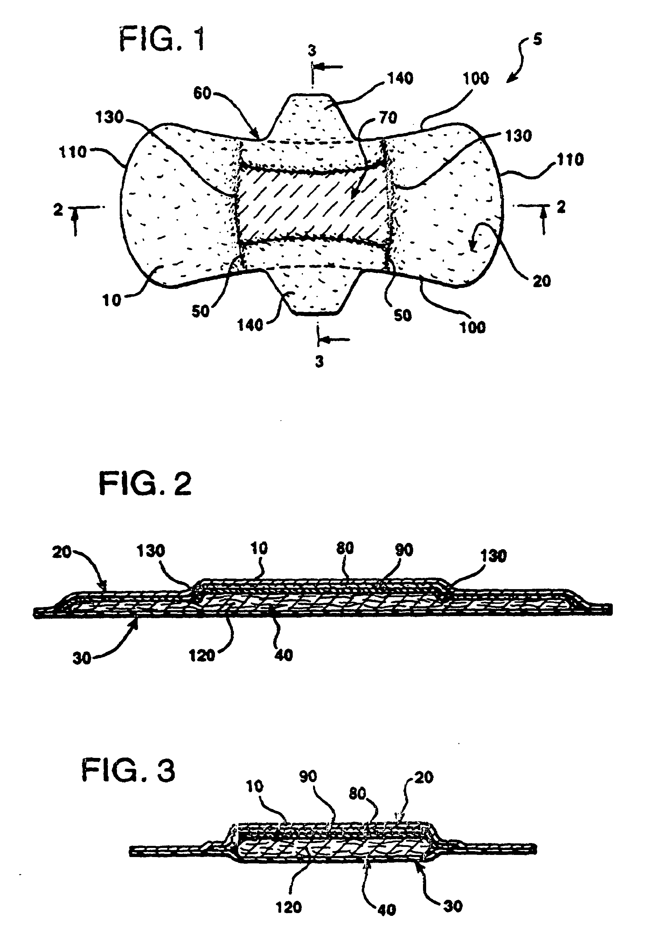

As illustrated in Figures 1-3, the absorbent articles **5** of this invention comprise the following elements:

a) a fluid-permeable cover **10** on the body-facing surface **20** of the article,
b) a fluid-impermeable backsheet **30**,
c) an absorbent core **40** between the cover and backsheet comprising wood pulp fluff and a center portion **60** containing a stabilizing element **70**, and preferential bending zones **50**.

Fluid-permeable cover sheet

The fluid-permeable cover sheet **10** may be composed of a woven or non-woven fibrous fabric or an apertured plastic sheet. The fibrous fabric may comprise cellulosic fibers such as cotton or rayon, polyolefin fibers such as polyethylene, polypropylene, polyester, and combinations thereof. It may be an entangled fabric, modified-entangled fabric, spun-bond fabric, melt-blown fabric, thermally bonded fabric or chemically bonded fabric.

Apertured plastic covers are well-known in the art. The cover may be selected from sheets known as Dri-weave (U.S. Patent No. 4,324,246), Reticulon® (U.S Patent No. 4,690,679) or Apex™ (U.S. Patent No. 5,567,376 and U.S. Patent No. 5,997,986.

Other suitable fluid-permeable cover sheets include absorbent, porous, dry-laid, nonwoven webs or scrim type materials such as those described in U.S. Pat. No. 4,880,419 to I.S. Ness and in U.S. Pat. No. 3,044,467 to Campau, in U.S. Pat. No. 3,463,154 to Hendricks and in U.S. Pat. No. 3,570,491 to Sneider.

Other suitable fluid-permeable cover sheets for use on the absorbent articles of the present invention include nonwovens made from hydrophobic fibers which have been coated with an adhesive or have been subjected to heat and/or pressure to fuse the individual fibers to each other such as those described in U.S. 4,795,455 to T. J. Luceri, in E.P. 354,502 to S. Cadieux, in E.P. 70,163 to A. T. Mays, and in U.S. 4,368,323 to R. P. James. Such materials tend to have only limited absorption thereby allowing passage to lower layers for absorption and retention. As a result, the fluid is wicked away from the body, leaving the surface of the body-contacting layer feeling drier to the touch.

The use of hydrophobic fibers for the body contacting, or cover, layer allows fluid to pass through to the absorbent layers beneath yet does not retain moisture on the surface layer, thus providing greater comfort since the wearer feels dry for a longer period of time. The desirability of such a feature has been disclosed in U.S. 3,838,692 to Levesque which describes a chemical method of providing porosity to hydrophobic materials.

Fluid-impermeable backsheet

The fluid-impermeable backsheet **30** may be a nonwoven or woven fabric treated to become impervious to fluid. Typically, though, the backsheet **30** is a plastic sheet comprised of polyethylene or polypropylene. Such layers are disclosed, for example, in U.S. 4,731,066 to Korpman.

Absorbent core

The absorbent core **40**, comprises wood pulp fluff, and may optionally contain other cellulosic material or commonly used absorbent materials, and is generally rectangular in shape, and is preferably hourglass shaped wherein a central region of the core, intermediate of the transverse ends of the core, has a width which is narrower than the width of the transverse ends. Such cores are taught, for example, in U.S. 4,552,618 to Kopolow and U.S. 4,536,432 to Holtman and in British patent 2,189,705 to Mesek. In a preferred embodiment, the absorbent core **40** contains additional pulp in the central region to raise this area in an upward, body contacting direction. Such additional thickness of pulp may be added between the absorbent core **40** and the stabilizing element **120**. This configuration will raise the stabilizing element **120**, and thus the body-facing surface of the absorbent article, closer to the body. Resistance to wet collapse may be enhanced by compressing the additional pulp or optionally the entire absorbent core prior to use.
Additionally, the absorbent core **40** may utilize a variety of fluid immobilizing materials, e.g., superabsorbing polymers or sphagnum moss, as a reservoir layer to increase fluid capacity or minimize the overall thickness of the absorbent article. Typically, these fluid immobilizing materials are not used alone, but in conjunction with cellulosic pulp fluff in order to provide extra absorbency for heavy fluid flow and to provide bulk to keep the article closer to the user's body. Such materials are taught by in U.S. 4,507,122 to Y. Levesque; in U.S. 4,494,963 to S. Dabi; in U.S. 4,880,419 to I. S. Ness; in U.S. 4,443,492 to J. Roller.
While such fluid immobilizing materials have the ability to absorb many times their weight in body fluid, their rate of absorption is relatively slow. Typically, a transfer layer **80** functions to quickly absorb fluid and hold it until a slower absorbing reservoir layer **90** can accept it. Thus, it is often preferred that a transfer layer **80** be incorporated between the cover sheet **10** and the upper body facing surface of the absorbent core **40**. The transfer layer **80** functions to rapidly draw fluid from the fluid-permeable cover sheet **10** and transport it to the absorbent stabilizing element and/or to that portion of the core into which the bulk of the fluid will eventually be absorbed, often referred to as a reservoir layer **90**. Thus, placement of the transfer layer **90** in the absorbent article would be between the cover sheet **10** and the reservoir layer **90**. Acceptable transfer layers **80** are those made from cellulosic materials, such as wood pulp, and an adhesive like binder. The basis weight of such materials would range from about 20-200 g/m$^2$. More preferably, they would range from about 50-150 g/m$^2$. Still more preferably, they would range from about 75-100 g/m$^2$. Still other suitable materials are discussed in U.S. patent application no. 08/075254, commonly assigned. Such materials, aside from those specifically disclosed in serial no. 08/075254, are well

Stabilizing element

The stabilizing element **120** is absorbent, resilient and stable, i.e. it maintains its shape in the wet state. The stabilizing element **120** should be resistant to wet-collapse in both the lateral or X-direction (herein defined as that direction transverse to the longitudinal edges **100**) and the Z-direction (herein defined as the direction normal to the cover sheet **10**). The wet collapse in the Z-direction should be less than 10% at 0.2 psi (1378.96 Pa) and less than 25% at 0.5 psi (3447.4 Pa). It should have a deformation measurement of less than 15% in the dry state and less than 35% in the wet state in accordance with the Wet and Dry Deformation Tests described in the Examples below.
Body-contact is important because it helps to inhibit expressed fluid from travelling along the body, e,g, the user's thighs, to soil the user's clothes. Since the stabilizing element **120** is resilient and does not collapse in a wet state, it thus enhances contact between the absorbent article **5** and the user's body at that point where fluid exits the body.
The resilient, absorbent stabilizing element **120** is preferably flexure resistant. Flexure resistance is generally measured by peak bending stiffness which is more fully discussed in U.S. 5,171,302 to Buell. The resilient, absorbent stabilizing element **120** of the present invention preferably has a peak bending stiffness of at least 250 grams, preferably greater than 400 grams, and most preferably greater than 600 grams.
The materials for use as the stabilizing element **120** are selected from the group consisting of sphagnum moss, calendered pulp, a composite of sphagnum moss and calendered pulp, and a composite of calendered pulp and superabsorbent material. These materials may comprise a single layer of the above materials, or may comprise multi-layered laminates. Alternatively, more than one stabilizing element may be used in the absorbent article on the invention. In a most preferred embodiment, the stabilizing element **120** comprises a calendered, multi-layer laminate of sphagnum peat moss which has been sandwiched between an upper and lower layer of pulp fibers.
The width of the stabilizing element **120** is considered to be a critical element of the present invention. The stabilizing element **120** must have a width sufficient to provide stability and resistance to lateral compression to the central portion of the absorbent article. Suitable widths are generally at least 0.5 inches (1.27 cm), and less than 1.75 inches (4.445 cm). It is preferred that the width of the stabilizing element **120** should generally approximate the dimensions of the labia majora so as to cover the area from which fluid will exit the body. Accordingly, the stabilizing element **120** may have a width in a range of from 0.5 to 1.75 inches (1.27 to 4.445 cm), preferably in a range of from 0.75 to 1.25 inches (1.905 to 3.175 cm), and most preferably, the stabilizing element **120** should have a width of about 1 inch (2.54 cm).

The length of the stabilizing element **120** is not, per se, critical to the invention, provided of course that it has a length sufficient to provide stability and resistance to lateral compression to the central portion of the absorbent article. Suitable lengths are generally at least about 2 inches (5.08 cm), and may be as long as the absorbent article. It is preferred that the length be less than that of the article **5** as a whole, and most preferably, the length and width of the stabilizing element **120** should conform generally to the longitudinal and lateral dimensions of the labia majora so as to cover the area from which fluid will exit the body. Accordingly, the stabilizing element **120** may have a length in a range of from 2 to 4 inches (5.08 to 10.16 cm), preferably in a range of from 2.5 to 3.5 inches (6.35 to 8.89 cm), and most preferably, the stabilizing element 120 should have a length of about 3 inches (7.62 cm) and a width of about 1 inch (2.54 cm). While the lengths of absorbent articles can vary widely, the foregoing preferred lengths generally correspond to less than about 80% of the article **5** length and, more preferably, between about 25-50% of the article **5** length.

The absorbent core **40** contains a central portion **60** which is located near the center of article **5**, which is defined by the portion between the longitudinal edges **100** and is preferably approximately midway between the transverse edges **110**. The central portion **60** would then be positioned, when in use, closest to the point where fluid exits the body. It is considered an important feature of the present invention that the stabilizing element **120** be located adjacent the central portion **60** and be slightly above a plane defined by the longitudinal edges **100** to keep in close contact with the body.

Preferential bending zones

The fluid-absorbent core contains two preferential bending zones. The stabilizing element is preferably in the center portion between the two preferential bending zones wherein the preferential bending zone(s) **50** are in a region outside the transverse ends of the stabilizing element **120**, and are preferably located at the end(s) **130** of the stabilizing element **120**. This allows the absorbent article **5** to cup at the front and back of the user while maintaining an essentially planar or flat configuration in the perineal area (when viewing the longitudinal edges of the article **5** from a longitudinal cross section - see for example Figure 2), thus allowing the stabilizing element **120** in the central portion **60** of the article **5** to conform to the shape of the user's body. More preferred are bending zones **50** which are transverse to the longitudinal axis of the absorbent article 5 and extend from one longitudinal side **100** to the other.

The bending location and profile of the napkin **5** should be such that the central portion **60** maintains an almost linear longitudinal profile when the ends of the napkin **5** are deflected by a specified amount. Example 3 below describes the testing for characterization of such preferential bending zones **50.** It is undesirable to have the central portion **60** cup or curve as the napkin **5** would move away from the user and protection would be sacrificed. The bending zone **50** may occur by a change in the flexibility of the napkin **5** along its length or it may occur due to a seam, space or embossment anywhere in those portions of the absorbent article **5** including the stabilizing element **120.**

An additional method of forming the preferential bending zones **50** involves changing the density of the absorbent article **5** such that the density of a stabilizing element **120** having a length which is less than that of the absorbent article is much greater than that of the absorbent core near the transverse ends **110**. Changing the density may occur by changing the thickness of the absorbent article **5** in the z-direction or by changing the basis weights of the materials in those parts of the absorbent article **5** adjacent the central portion **60.**

The absorbent article **5** may also be pre-shaped such that the napkin **5**, prior to use, has a bend located at the preferential bending zones. Such pre-shaping may be accomplished, for example by placing elastic in the flanged side margins of longitudinal edges **100** of the napkin **5** as disclosed in commonly assigned U.S. Patent Application Serial No. 07/766699, and which is hereby incorporated herein by reference in its entirety.

The absorbent articles of this invention optionally, but preferably, contain attachment tabs and/or side cuffs along the longitudinal sides of the absorbent core. These attachment tabs are preferably of dimensions such that their longitudinal edges are shorter than the longitudinal dimensions of the stabilizing element so as not to impede the article's bending along the preferential bending zones located adjacent the stabilizing element.

Attachment tabs **140** possess adhesive means to hold the article **5** securely to the crotch portion of a user's undergarment so that the stabilizing element **120** maintains intimate contact with the body. The attachment tabs **140** also serve to prevent the user's undergarment from contacting the longitudinal sides **100** of the central portion **60** by restraining the side edges of the crotch portion of the user's undergarment. It is preferred, however, that the line of juncture of the attachment tabs **140** not extend beyond the preferential bending zones **50** as this would inhibit bending of the article **5**. The attachment tabs **140** may be attached to the longitudinal sides **100** of the article **5** or they may be located on the garment facing side inward from the longitudinal edges as disclosed more fully in U.S. 4,900,320 to McCoy, which incorporated herein by reference in its entirety.

It is preferable that the line of juncture between the tabs **140** and the napkin **5** be less than 7 inches (17.78 cm). It is more preferable that the line of juncture be between 3 and 6 inches (7.62 and 15.24 cm).

It is also preferred that the absorbent article **5** be hourglass shaped having arcuate lateral sides with the front and rear portions wider than the central portion **60.** This configuration will also help to stabilize the article **5** by inhibiting shifting from front to back.

In order to demonstrate the features of this invention, the following non-limiting examples below are submitted. In all instances the napkins manufactured for testing in the examples utilized a perforated plastic film (PE/EVA or PE) for the cover (either 0.54 or 1.0 oz/yd$^2$ (18.3 or 33.9 gim2)), a transfer layer of 90 g/m$^2$ airlaid, latex bonded pulp fabric, a wetlaid cellulosic absorbent having a basis weight of 325 g/m$^2$, a core of wood pulp fluff underneath (garment side) of a sphagnum moss absorbent board, and a PE/EVA film barrier layer.

Example 1 - Z-direction Deformation Test

The test described below was designed to simulate the z-direction pressure exerted on a sanitary napkin by a wearer's body. Ideally, the napkin should not collapse in the z-direction when exposed to body fluids, such as menstrual fluid, and body pressure. As demonstrated below, conventional napkins are subject to such collapse.

Ten napkins were prepared according to the teachings of the specification and compared to ten control napkins, commercially available from Proctor & Gamble under the trademark ALWAYS PLUS MAXI. Each sample was conditioned at 70° $\pm$ 2° F (21.1° $\pm$ 1.1° C) and 50 $\pm$ 2% RH for 24 hours prior to the test. Such conditions were maintained throughout the testing. The test is further described below. The discussion will detail how each sample was tested.

An AMES gauge #91-013, with a 1.125 inch (1.8575 cm) diameter foot weighing 12.7 grams, was calibrated using standard blocks under a standard 56.7 gram weight (the total weight corresponds to a pressure of 0.15 psi (1034.22 Pa)). The foot of the gauge was raised and the sample was placed on the anvil. The foot was then lowered gently onto the center of the sample and allowed to remain for 15 seconds before taking a reading. This is the initial thickness $W_1$.

The weight on the AMES gauge was then increased to 85.3 grams (this plus the weight of the foot corresponds to a pressure of 0.22 psi (1516.856 Pa)). The thickness of the sample was again measured and noted as thickness $W_2$.

The center of the sample was marked where the foot of the gauge was resting. The sample was removed and cc of synthetic menstrual fluid (SMF) was introduced to the marked area using a syringe. The fluid was added slowly enough so that it did not spill outside the marked area. The sample was then immediately placed on the AMES gauge with a weight of 85.3 grams. The thickness of the sample was noted then and for each minute thereafter until there was no change in the thickness of the sample. (In all samples, the thickness ceased to change after 4 minutes). This final thickness was recorded as $W_1$.

All pressure was then removed from the sample by removing it from the AMES gauge, and it was allowed to stand for 15 minutes. After 15 minutes the sample was placed in the AMES gauge and a weight of 56.7 grams was introduced. The thickness under this pressure was recorded as $W_4$.

From these four readings it is possible to calculate the % reduction in thickness normal use (%RT) by the following equation:

$$\%RT = (W_2 - W_3)/W_2 \times 100$$

From these four readings it is also possible to calculate the % delayed reduction in thickness (%DR) by the following equation:

$$\%DR = (W_1 - W_4)/W_1 \times 100$$

Ten additional napkins were prepared according to the teachings of the specification and compared to an additional ten control napkins, commercially available from Proctor & Gamble under the trademark ALWAYS PLUS MAXI. The procedure described above was repeated, except that the test pressure, $W_2$ was measured using a 226.8 gram weight (corresponding to a total pressure of 0.53 psi (3654.244 Pa)).

Table 1 shows the results obtained under a test pressure of 0.22 psi (1516.856 Pa). Table 2 shows the results obtained under a test pressure of 0.53 psi (3654.244 Pa).

TABLE 1 TEST PRESSURE AT 0.22 PSI (1516.856 Pa)

|  | Test | | Control | |
|---|---|---|---|---|
|  | %RT | %DR | %RT | %DR |
|  | 7.496 | 4.210 | 30.769 | 30.320 |
|  | 7.192 | 5.980 | 29.725 | 28.012 |
|  | 3.580 | -1.300 | 26.375 | 26.179 |
|  | 5.187 | -1.408 | 29.566 | 23.994 |
|  | 5.650 | 1.284 | 24.296 | 21.407 |
|  | 3.571 | -3.102 | 24.160 | 23.566 |
|  | 10.069 | 4.232 | 23.414 | 24.606 |
|  | 6.666 | 0.160 | 24.437 | 23.557 |
|  | 4.566 | -2.215 | 23.455 | 22.606 |
|  | 6.593 | -0.356 | 23.411 | 22.186 |
| Mean | 6.057 | 0.747 | 25.961 | 24.643 |
| S.D. | 1.988 | 3.090 | 2.945 | 2.776 |

TABLE 2 TEST PRESSURE AT 0.53 PSI (3654.244 Pa)

|  | Test | | Control | |
|---|---|---|---|---|
|  | %RT | %DR | %RT | %DR |
|  | 24.750 | 23.279 | 36.538 | 32.132 |
|  | 26.615 | 22.684 | 37.681 | 31.288 |
|  | 28.740 | 27.632 | 36.501 | 35.453 |
|  | 27.490 | 25.333 | 34.904 | 34.407 |
|  | 26.556 | 27.682 | 35.088 | 27.190 |
|  | 23.390 | 23.611 | 38.021 | 37.215 |
|  | 19.408 | 17.139 | 36.678 | 38.587 |
|  | 21.839 | 17.949 | 37.343 | 29.566 |
|  | 18.868 | 15.120 | 35.528 | 34.153 |
|  | 22.901 | 20.886 | 39.082 | 34.894 |
| Mean | 24.056 | 22.131 | 36.736 | 33.489 |
| S.D. | 3.370 | 4.324 | 1.335 | 3.476 |

Example 2 - X-direction Deformation Test

The objective of this test is to determine the deformation resistance of a sanitary napkin or any absorbent article in X-direction (the direction transverse to the longitudinal axis across the plane of the napkin) in terms of a loss in napkin width in both wet and dry states. Generally the tests consists of holding a napkin around the inner cylinder of an Instron deformation test apparatus. It is compressed and then allowed to recover from its deformation for 2 cycles. The loss in width as a result of compression and relaxation is a measure of the deformation resistance of the napkin in X-direction.

The apparatus used for the test was: 1. Instron Model 1122 Universal Tester; 2. a custom jig (Figure 4) with an 8 inch diameter cylinder and upper and lower jaws which are shaped to resemble a human thigh; 3. 100 Kg load cell; 4. vernier caliper; 5. 25cc graduated cylinder; 6. plexiglas plate (0.511 (1.3 cm) thick ) with an oblong center opening of 0.75" x 1.50" (1.778 x 3.81 cm); 7. a 3 inch (7.62 cm) wide knitted fabric (45% polyester/45% cotton/10% Lycra spandex) which simulates an undergarment crotch; 8.

Ten napkins were prepared according to the teachings of the specification and ten control napkins, commercially available from Proctor & Gamble under the trademark ALWAYS MAXI PLUS, were purchased. Each sample was conditioned at 70° ± 2° F (21.1° ± 1.1° C) and 50 ± 2% RH for 24 hours prior to the test. Such conditions were maintained throughout the testing. The test is further described below. The discussion will detail how each sample was tested.

The gap between the upper and lower compression surfaces of the custom jig was set at 0.8 inch (2.032 cm). The return limit on the Instron console was set at 2.2 inches (5.588 cm). The crosshead speed was set at 5 in./minute (12.7 cm/min). The test direction was set to "up" and was pressed so that the crosshead starts moving, stopping when reaching a 3 inch (7.62 cm) gap spacing (0.8 Inch (2.032 cm) initial gap + 2.2 inch (5.588 cm) crosshead travel).

The instrument was then calibrated by setting the load scale dial to 2 Kg and zeroing the pen on the chart. A 1 Kg weight was then placed on the lower cylinder ring jaw and pen was adjusted using the "calibration knob" so that the pen rested on the 1 Kg line. The 1 Kg weight was then removed. If the pen returns back to the zero position on the chart, it means the instrument has been calibrated. If needed, the "balance knob" is used to bring the pen back to zero.

The 3 inch (7.62 cm) wide fabric was cut such that the grain of the fabric was perpendicular to the direction of travel. A 4 inch (10.16 cm) long test sample element was cut from the center of each sanitary napkin to be tested. The width of each test sample was the width of the absorbent system in the napkin. The width of the test element was measured using a Vernier caliper and was recorded as initial width $L_1$.

The release paper, which is on the garment facing side, was removed from the sample. The sample was then placed with the garment facing side down into the center of the inner surface of the loop to be formed on the Jig with the side edges of the fabric enveloping the sample edges. The sample was secured on the fixture in such a way that it was in direct contact with the jig surface and centered between the curved jaws.

The cycle counter was set to "2" so that the test sample could be compressed for 2 cycles. The return limit on the console was set to "2.25." The maximum and minimum limits were set to "2.20" and "0.00" respectively. The test direction was set to "down", and the crosshead speed was set to 5 in./minute (12.7 cm/min.).

The Instron was started. After the crosshead had compressed the sample for 2 cycles and stopped, the sample was is removed and the width again measured and is recorded as final width $L_2$.

Napkins were also tested in the wet state by preparing the samples as described above. However, prior to positioning the sample on the jig, SMF was applied to the center of the test element. This was done by placing the plexiglas plate over the center of the sample. The SMF cc of fluid was poured from the graduated cylinder into the oblong opening of the plate placed on to the sample. Once the fluid was absorbed, the sample was allowed to remain at test conditions for 1 minute and then $L_1$ and $L_2$ measured as described above.

The percentage of amount of x-direction deformation (%XD) was calculated as follows:

$$\%XD = (L_1 - L_2)/L_1 \times 100$$

Table 3 shows the results obtained in the dry state. Table 4 shows the results obtained in the wet state.

TABLE 3

| % LOSS IN WIDTH IN X-DIRECTION IN DRY STATE | | | | | |
|---|---|---|---|---|---|
| TEST | | | CONTROL | | |
| L1 | L2 | %XD | L1 | L2 | %XD |
| 1.99 | 1.70 | 14.57 | 1.65 | 1.44 | 12.73 |
| 1.89 | 1.61 | 14.81 | 1.72 | 1.36 | 20.93 |
| 1.91 | 1.71 | 10.47 | 1.75 | 1.30 | 26.47 |
| 1.96 | 1.74 | 11.22 | 1.72 | 1.40 | 18.60 |
| 1.91 | 1.78 | 6.80 | 1.70 | 1.49 | 13.53 |
| 1.83 | 1.60 | 12.57 | 1.78 | 1.39 | 21.91 |
| 1.84 | 1.65 | 10.33 | 1.71 | 1.34 | 21.64 |
| 1.81 | 1.68 | 7.18 | 1.74 | 1.46 | 16.09 |
| 1.83 | 1.65 | 9.84 | 1.65 | 1.41 | 14.54 |
| 1.82 | 1.65 | 9.34 | 1.74 | 1.46 | 16.09 |

(continued)

| % LOSS IN WIDTH IN X-DIRECTION IN DRY STATE | | | | | |
|---|---|---|---|---|---|
| TEST | | | CONTROL | | |
| L1 | L2 | %XD | L1 | L2 | %XD |
| Mean =<br>S.D. = | | 10.71<br>2.71 | | | 18.24<br>4.42 |

TABLE 4

| % LOSS IN WIDTH IN X-DIRECTION IN WET STATE | | | | | |
|---|---|---|---|---|---|
| TEST | | | CONTROL | | |
| L1 | L2 | %XD | L1 | L2 | %XD |
| 1.86 | 1.27 | 31.72 | 1.69 | 0.98 | 42.01 |
| 1.84 | 1.23 | 33.15 | 1.69 | 0.93 | 44.97 |
| 1.90 | 1.32 | 30.53 | 1.65 | 0.99 | 40.00 |
| 1.83 | 1.28 | 30.05 | 1.67 | 0.98 | 41.32 |
| 1.87 | 1.33 | 28.88 | 1.68 | 1.06 | 36.90 |
| 1.80 | 1.26 | 30.00 | 1.64 | 1.00 | 39.02 |
| 1.85 | 1.28 | 30.81 | 1.64 | 0.87 | 46.95 |
| 1.78 | 1.23 | 30.90 | 1.66 | 0.93 | 43.98 |
| 1.83 | 1.28 | 30.05 | 1.67 | 0.94 | 43.71 |
| 1.83 | 1.23 | 32.79 | 1.61 | 0.93 | 42.24 |
| Mean =<br>S.D. = | | 30.89<br>1.32 | | | 42.11<br>2.97 |

As is clear from both Table 3 and Table 4, the napkin as described in this invention offers more resistance to deformation in X-direction as compared to conventional napkins. The conventional napkins, which mainly consist of pulp, undergo deformation in X-direction in both dry as well as wet states. This phenomenon, called bunching, reduces the effective surface area of the absorbent system available for the capture of bodily fluid. The severe deformation in X-direction, therefore, potentially can lead to higher chances of undergarment staining.

Example 3 - Bending Test

Bending zones were created by embossing transversely across the samples at each end of the stabilizing element (defined as the ends of the sphagnum board) and by decreasing the thickness of the pads both fore and aft of the stabilizing element. This example consists of two parts. The first part demonstrates where the preferential bending zones are located upon application of force along the longitudinal axis. The second part demonstrates the difference in bending resistance between a tab with a 2.75 inch (6.985 cm) line of juncture between the tab and the napkin and a tab with a five inch line of juncture between the tab and the napkin.

**Claims**

1.  An absorbent article (5) having longitudinal sides (100), transverse ends (110), a body-facing surface (20) and a garment-facing surface, said article (5) comprising:

   (a) a fluid-permeable cover (10) on said body-facing surface (20);
   (b) a fluid-impermeable barrier (30) on said garment-facing surface;
   (c) a fluid-absorbent core (40) containing wood pulp fluff between the fluid-permeable cover (10) and the fluid impermeable barrier (30), said fluid-absorbent core (40) having a central region (60), transverse ends (130) and a thickness of at least about 0.20 inches (0.508 cm); and
   (d) a stabilising absorbent element (120) adjacent an upper portion of the central region (60) of the absorbent core (40), wherein the stabilising element (120) is capable of absorbing fluids and remaining stable when wet,

**characterised in that** the fluid-absorbent core (40) contains two preferential bending zones (50) in a region outside the transverse ends of the stabilizing element (120); the stabilising element (120) has a lateral width in a range of from at least 0.5 inches (1.27 cm) to less than 1.75 inches (4.445 cm) and is substantially planar; and, in use, bending a the preferential bending zones (50) allows the absorbent article (5) to cup while the stabilising element (120) maintains the absorbent article (5) in an essentially planar configuration in the perineal area; and wherein the stabilising element (120) is sphagnum moss, calendered pulp, a composite of sphagnum moss and calendered pulp or a composite of calendered pulp and superabsorbent material.

2. The absorbent article (5) of claim 1 wherein the absorbent core (40) has an hour glass shape wherein the central region (60) of the absorbent core (40) has a narrower lateral width than the transverse ends (130) of the absorbent core.

3. The absorbent article (5) of claim 1 or claim 2 wherein the stabilising element (120) comprises a multi-layer laminate of sphagnum moss and wood pulp fibers.

4. The absorbent article (5) of any one of claims 1 to 3 wherein the stabilising element (120) has a length in a range of from 2 to 4 inches (5.08 to 10.16 cm), preferably of from 2.5 to 3.5 inches (6.35 to 8.89 cm), and most preferably has a length of about 3 inches (7.62 cm).

5. The absorbent article (5) of claim 4 wherein the stabilising element (120) has a width of about 1 inch (2.54 cm).

6. The absorbent article (5) of any one of claims 1 to 5 wherein the stabilising element (120) has a peak bending moment of at least about 250 grams, preferably of greater than about 400 grams, most preferably of greater than about 600 grams.

7. The absorbent article (5) of any one of claims 1 to 6 wherein the stabilising element (120) has a resistance to wet collapse of less than about 10% at 0.2 psi (1378.96 Pa), preferably of less than about 25% at 0.5 psi (3447.4 Pa).

**Patentansprüche**

1. Absorbierendes Produkt (5) mit in Längsrichtung verlaufenden Seiten (100), in Querrichtung verlaufenden Enden (110), einer körperseitigen Oberfläche (20) und einer kleidungsseitigen Oberfläche, wobei das Produkt (5) umfasst:

a) eine fluiddurchlässige Abdeckung (10) auf der körperseitigen Oberfläche (20),
b) eine fluidundurchlässige Barriere (30) an der kleidungsseitigen Oberfläche;
c) einen Fluid absorbierenden Kern (14), welcher Zellstofffllocken enthält, zwischen der fluiddurchlässigen Abdeckung (10) und der fluidundurchlässigen Barriere (30), wobei der Fluid absorbierende Kern (40) einen Zentralbereich (60), in Querrichtung verlaufende Enden (130) und eine Dicke von mindestens ungefähr 0,20 Zoll (0,508 cm) aufweist; und
d) ein stabilisierendes absorbierendes Element (120) in der Nähe eines oberen Teils des Zentralbereiches (60) des absorbierenden Kerns (40), wobei das stabilisierende Element (120) in der Lage ist, Fluide zu absorbieren und in nassem Zustand stabil zu bleiben,

**dadurch gekennzeichnet, dass** der Fluid absorbierende Kern (40) zwei Vorzugs-Biegezonen (50) in einem Bereich außerhalb der in Querrichtung verlaufenden Enden des stabilisierenden Elements (120) enthält; das stabilisierende Element (120) eine Breite in Querrichtung in einem Bereich von mindestens 0,5 Zoll (1,27 cm) bis zu weniger als 1,75 Zoll (4,445 cm) aufweist und im wesentlichen eben ist; und bei der Benutzung ein Verbiegen der Vorzugs-Biegezonen (50) es dem absorbierenden Produkt gestattet (5), sich zu wölben, wobei das stabilisierende Element (120) das absorbierende Produkt (5) in einer im wesentlichen ebenen Konfiguration in dem perinealen Bereich hält; und wobei das stabilisierende Element (120) Sphagnum, kalandrierter Zellstoff, ein Verbundstoff aus Sphagnum und kalandriertem Zellstoff oder ein Verbundstoff aus kalandriertem Zellstoff und superabsorbierendem Material ist.

2. Absorbierendes Produkt (5) nach Anspruch 1, wobei der absorbierende Kern (40) eine Sanduhrform besitzt, wobei der zentrale Bereich (60) des absorbierenden Kerns (40) eine geringere seitliche Breite als die in Querrichtung verlaufenden Enden (130) des absorbierenden Kerns (40) aufweist.

**3.** Absorbierendes Produkt (5) nach Anspruch 1 oder 2, wobei das stabilisierende Element (120) ein Laminat mit mehreren Schichten aus Sphagnum und Zellstofffasern enthält.

**4.** Absorbierendes Produkt (5) nach einem der Ansprüche 1 bis 3, wobei das stabilisierende Element (120) eine Länge in einem Bereich von 2 bis 4 Zoll (5,08 bis 10,16 cm), vorzugsweise in einem Bereich von 2,5 bis 3,5 Zoll (6,35 bis 8,89 cm) aufweist und höchst bevorzugt eine Länge von etwa 3 Zoll (7,62 cm) besitzt.

**5.** Absorbierendes Produkt (5) nach Anspruch 4, wobei das stabilisierende Element (120) eine Breite von ungefähr 1 Zoll (2,54 cm) aufweist.

**6.** Absorbierendes Produkt (5) nach einem der Ansprüche 1 bis 5, wobei das stabilisierende Element (120) ein Spitzenbiegungsmoment von mindestens ungefähr 250 g, vorzugsweise mehr als ungefähr 400 g und höchst vorzugsweise mehr als ungefähr 600 g aufweist.

**7.** Absorbierendes Produkt (5) nach einem der Ansprüche 1 bis 6, wobei das stabilisierende Element (120) eine Festigkeit gegenüber einem Kollaps in nassem Zustand von weniger als etwa 10% bei 0,2 psi (1378,96 Pa), vorzugsweise von weniger als etwa 25% bei 0,5 psi (3447,4 Pa) aufweist.


**Revendications**

**1.** Article absorbant (5) comportant des côtés longitudinaux (100), des extrémités transversales (110), une surface côté corps (20) et une surface côté vêtement, ledit article (5) comprenant :

   a) une couche de couverture perméable aux fluides (10) sur ladite surface côté corps (20) ;
   b) une barrière imperméable aux fluides (30) sur ladite surface côté vêtement ;
   c) un noyau absorbant les fluides (40) contenant un duvet de pâte de bois entre la couche de couverture perméable aux fluides (10) et la barrière imperméable aux fluides (30), ledit noyau absorbant les fluides (40) comportant une région centrale (60), des extrémités transversales (130) et une épaisseur d'au moins environ 0,20 pouce (0,508 cm) ; et
   d) un élément absorbant stabilisant (120) adjacent à une partie supérieure de la région centrale (60) du noyau absorbant (40), dans lequel l'élément stabilisant (120) est capable d'absorber les fluides et de rester stable à l'état mouillé,

   **caractérisé en ce que** le noyau absorbant les fluides (40) contient deux zones de cintrage préférentielles (50) dans une région extérieure aux extrémités transversales de l'élément stabilisant (120) ; l'élément stabilisant (120) possède une largeur latérale dans une plage d'au moins 0,5 pouce (1,27 cm) à moins de 1,75 pouces (4,445 cm) et est sensiblement plan ; et, durant l'utilisation, le cintrage au niveau des zones de cintrage préférentielles (50) permet à l'article absorbant (5) de former un creux alors que l'élément stabilisant (120) maintient l'article absorbant (5) dans une configuration essentiellement plane dans la région périnéale ; et dans lequel l'élément stabilisant (120) est de la sphaigne, de la pâte calandrée, un composite de sphaigne et de pâte calandrée ou un composite de pâte calandrée et de matériau superabsorbant.

**2.** Article absorbant (5) selon la revendication 1, dans lequel le noyau absorbant (40) possède une forme de sablier, dans lequel la région centrale (60) du noyau absorbant (40) possède une largeur latérale plus étroite que les extrémités transversales (130) du noyau absorbant (40).

**3.** Article absorbant (5) selon la revendication 1 ou la revendication 2, dans lequel l'élément stabilisant (120) comprend un stratifié multicouche de sphaigne et de fibres de pâte de bois.

**4.** Article absorbant (5) selon une quelconque des revendications 1 à 3, dans lequel l'élément stabilisant (120) possède une longueur dans la plage de 2 à 4 pouces (5,08 à 10,16 cm), de préférence dans une plage de 2,5 à 3,5 pouces (6,35 à 8,89 cm) et, idéalement, possède une longueur d'environ 3 pouces (7,62 cm).

**5.** Article absorbant (5) selon la revendication 4, dans lequel l'élément stabilisant (120) possède une largeur d'environ 1 pouce (2,54 cm).

**6.** Article absorbant (5) selon une quelconque des revendications 1 à 5, dans lequel l'élément stabilisant (120) possède un moment de cintrage maximal d'au moins environ 250 grammes, de préférence supérieur à environ 400 grammes, idéalement supérieur à environ 600 grammes.

7. Article absorbant (5) selon une quelconque des revendications 1 à 6, dans lequel l'élément stabilisant (120) possède une résistance à la déformation à l'état mouillé inférieure à environ 10 % à 0,2 psi (1378,96 Pa), de préférence inférieure à environ 25 % à 0,5 psi (3447,4 Pa).

# FIG. 1

# FIG. 2

# FIG. 3

**EP 0 847 265 B2**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 091412 A
- US 4770657 A
- WO 8804547 A
- US 4195634 A
- US 4405326 A
- US 4217901 A
- EP 0335252 A
- EP 0335253 A
- US 4490147 A
- US 4631062 A
- US 4804380 A
- US 2662527 A
- US 3406689 A
- US 4433972 A
- US 4425130 A
- WO 9207535 A
- US 3397697 A
- US 4285343 A
- US 4589876 A
- US 4687478 A
- EP 0426235 A
- US 4608407 A
- US 4911701 A
- US 4900320 A
- US 4324246 A
- US 4690679 A
- US 5567376 A
- US 5997986 A
- US 4880419 A
- US 3044467 A
- US 3463154 A
- US 3570491 A
- US 4795455 A
- US 4368323 A
- US 3838692 A
- US 4731066 A
- US 4552618 A
- US 4536432 A
- GB 2189705 A
- US 4507122 A
- US 4494963 A
- US 4443492 A
- US 075254 A
- US 5171302 A
- US 766699 A